# EUROPEAN PATENT APPLICATION

(11) **EP 3 753 488 A1**
(43) Date of publication of application: **23.12.2020**
(21) Application number: 19181417.7
(22) Date of filing: 20.06.2019
(51) Int. Cl.: A61B 5/06, A61B 5/053, A61B 5/107, A61B 5/00, A61B 5/042, A61B 5/1495

(54) **SYSTEM AND METHOD FOR DETERMINING A VENTRICULAR GEOMETRY, ELECTROANATOMICAL MAPPING SYSTEM COMPRISING SUCH A SYSTEM, AND METHOD FOR DETERMINING A VENTRICULAR GEOMETRY**

(71) Applicant: VascoMed GmbH, 79589 Binzen (DE)
(72) Inventor: Codreanu, Ovidiu, 10551 Berlin (DE); Stump, Joachim, 12621 Berlin (DE)
(74) Representative: Galander, Marcus

(57) **Abstract**

The disclosure relates to a system (1) and a method for determining a heart chamber geometry. The system (1) comprises: a catheter (2) being configured to be introduced in a heart chamber of a patient, the catheter (2) comprising a plurality of catheter electrodes (21); a position determination subsystem (4) being configured to determine a position of each of the catheter electrodes (21) by means of multiple localization electrodes (41) that are configured to be arranged on the patient's body; a wall contact detection subsystem (5) being configured to determine for each of the catheter electrodes (21) whether the catheter electrode (21) is in contact with a heart chamber wall of the heart chamber by measuring at least one of a complex impedance, an amount of a complex impedance, and a phase of a complex impedance; and a modelling subsystem (6) being configured to create a model of the heart chamber geometry by selecting, among a set of positions of the catheter electrodes (21) as acquired by means of the position determination subsystem (4), positions in which the catheter electrodes (21) are in contact with the heart chamber wall as determined by means of the wall contact detection subsystem (5).

## Description

The present disclosure relates to a system for determining a heart chamber geometry as well as to a method for determining a heart chamber geometry using such a system. The present disclosure also relates to an electroanatomical mapping system.

Systems for determining a heart chamber geometry can be used, for example, for the purpose of providing a 3D navigation support during an intracardiac ablation treatment.

Intracardiac ablation is an alternative method to the non-drug treatment of cardiac arrhythmias. Ablation procedures are increasingly carried out with 3D navigation support. For an accurate navigation, the 3D systems require a geometric model of the examined heart chamber (e.g. the ventricle). This model is usually determined by guiding a catheter within the chamber to many different locations and locating them simultaneously. While the user moves the catheter inside the heart chamber, a catheter tip reaches different locations within the heart chamber. From the determined positions of the catheter tip, a closed surface is modeled by appropriate software, which should ideally reflect the shape of the endocardium. In other words, the different localized positions of the catheter tip span a closed surface that approximates the geometry of the heart chamber, which is later used for the actual 3D navigation.

During the conventional procedure described above, the user determines, based on his experience, through tactile feedback on the handle of the catheter, whether he has reached the inner surface of the heart wall. A contact force may also be indicated by a "Contact Force" unit. In some known applications, the user's general knowledge of heart anatomy may be supplemented by IEGM (Intracardiac Electrogram) data an image acquired by CT, MRI or 3D angiometry. Sometimes, a contact impedance at the tip of the ablation may be measured in addition. For example, the impedance may be displayed at an RF generator during the procedure.

The conventional procedure has several drawbacks. For example, the user must exert some pressure on the catheter tip to tactilely detect whether it has reached the heart wall. Depending on the user's experience, this may pose a risk to the patient. Apart from tactile feedback, the user does not receive reliable information as to whether the catheter has been brought in contact with the endocardium.

As a result, there may be locations, in which the generated model clearly does not correspond to the real heart chamber geometry. For instance, a patient-specific heart anatomy with an atypical indentation on the inner surface of the heart, which was not touched by direct wall contact, could be inadvertently made invisible within the generated model by an interpolation surface between touched points, unless an image of CT, MRI or 3D angiography is displayed in parallel.

The procedure is time-consuming because the user has to make sure that the correct geometry has been captured as accurately as possible. Often, the user has to touch certain regions repeatedly or in a particularly fine grid. Due to the required time, the geometry is in many cases not determined repeatedly during an ablation procedure, even if it may be no longer up-to-date for various reasons (drift, movement of the patient, etc.).

Further, the user usually needs to verify with X-ray images to what extent the current iteration in the generation of the model has approached reality. This leads to increased exposure of both the patient and the user to the radiation.

If a special ablation catheter with a force sensor is used, the contact pressure for the tip can be displayed. A dedicated force evaluation unit is required for this. Further, this method cannot be applied with common diagnostic catheters or with catheters having several catheter electrodes.

If support by IEGM monitoring is additionally provided, the user can decide based on his experience whether the heart wall has been touched or not. This method is not practicable for catheters having several catheter electrodes because the synchronization of catheter movements with the monitoring of the 3D model and with the amplitude and shape of several IEGM signals poses a virtually insurmountable challenge. This method cannot provide certainty that the catheter electrodes have actually come into contact with the endocardium.

The places where the wall contact has already been evaluated by these common methods are not easily recognizable in the 3D model. There is thus a high probability that they will be unnecessarily touched again, which requires additional time. Specifically, it may be unclear, which portions of the generated area actually correspond to the endocardial geometry as determined by direct probing and which proportions are merely interpolated areas between the probed points.

In summary, the known procedures for determining a heart chamber geometry described above are relatively time-consuming for the user and their results may be relatively inaccurate.

Document US 2018/0014751 A1 discloses computer-implemented systems and methods for generating smoothed images of an elongate medical device, such as, for example, a catheter within a body.

Document US 7,263,397 B2 describes the use of catheters to create an image of the interior of the heart, wherein electrophysiology information collected from the catheters is presented to a physician user to guide a therapy or diagnosis.

It is an object to provide improved technologies for determining a heart chamber geometry. For example, it is desirable to provide for a more reliable and accelerated acquisition of a heart chamber geometry in 3D navigation systems, e g., in the context of an ablation procedure. Further, it is desirable to improve the reliability of electroanatomical mapping (EAM).

According to a first aspect, a system for determining a heart chamber geometry according to claim 1 is provided.

The system comprises: a catheter being configured to be introduced in a heart chamber of a patient, the catheter comprising a plurality of catheter electrodes; a position determination subsystem being configured to determine a position of each of the catheter electrodes by means of multiple localization electrodes that are configured to be arranged on the patient's body; a wall contact detection subsystem being configured to determine for each of the catheter electrodes whether the catheter electrode is in contact with a heart chamber wall of the heart chamber by measuring at least one of a complex impedance, a magnitude of a complex impedance, and a phase of a complex impedance; and a modelling subsystem being configured to create a model of the heart chamber geometry by selecting, among a set of positions of the catheter electrodes as acquired by means of the position determination subsystem, positions in which the catheter electrodes are in contact with the heart chamber wall as determined by means of the wall contact detection subsystem.

The heart chamber may refer to the left ventricle, the right ventricle, the left atrium, the right atrium or any other chamber of the heart. The heart chamber geometry may refer to the geometry of a ventricle (left or right ventricle), the geometry of an atrium (left and right atrium) or any other heart chamber geometry.

Hence, it is proposed to use a contact impedance directly measured on multiple catheter electrodes for reliably determining a contact with a heart chamber wall and to simultaneously locate the multiple catheter electrodes by means of multiple localization electrodes. The results of these measurements are then combined to quickly generate a reliable geometrical model of the heart chamber consistent with its actual shape without the need for additional image data from CT, MRI, or 3D angiography.

In accordance with some embodiments, the catheter comprises at least 4 catheter electrodes, such as at least 10 catheter electrodes, such as at least 30 catheter electrodes, or even at least 60 catheter electrodes. For example, in a preferable embodiment, 64 catheter electrodes are provided. A catheter having multiple electrodes can speed up the procedure of determining the heart chamber geometry.

For example, the catheter electrodes may be configured as ring electrodes and/or tip electrodes, which may be arranged, e. g., in the vicinity of a distal end of a catheter. Such kinds of catheter electrodes are, as such, known in the art and they will therefore not be described in greater detail in the following. In accordance with the present disclosure, when creating the model of the heart chamber geometry, the impedance for all catheter electrodes can be evaluated. As a result, the contact of all catheter electrodes to the heart wall may be determined and may be taken into account in the model creation. This is beneficial for the accuracy of the resulting 3D model. If used for 3D navigation an during ablation therapy, a more reliably proximity can thus be provided to the user during ablation because the geometric heart chamber model created by means of the present disclosure is very close to reality.

In an embodiment, the wall contact detection subsystem comprises a reference electrode that is configured to be arranged on or inside the patient's body. The reference electrode may be, for example, a surface electrode or an intracardiac electrode. In another embodiment, a virtual potential formed by combining several signals acquired on the patient may be used as a reference potential for the contact detection.

Further, in an embodiment, the wall contact detection subsystem comprises a signal generator being configured to feed an electrical probe signal, such as a current signal, to each of the catheter electrodes. For example, the electrical probe signal may be an alternating current signal having a frequency in the range from 10 kHz to 50 kHz. In a preferred embodiment, the frequency of the electrical probe signal is 25±2 kHz.

In a variant embodiment, the signal generator is configured to feed the electrical probe signal to the catheter electrodes in a time multiplexed manner, wherein a multiplexing frequency may be at least 10 Hz, such as at least 2000 Hz. In a preferred embodiment, the multiplexing frequency is 22 Hz. This is to say that, on a recurring basis, the multiple catheter electrodes may sequentially receive the probe signal, such that each of the control electrodes receives the probe signal at least 10 times per second, such as at least 2000 times per second, e. g., 22 times per second. Such multiplexing frequencies are sufficiently large so as to resolve the heartbeat of the patient. For example, this allows for the detection of an intermittent contact of a catheter electrode with the heart chamber wall, which results from the movement of the heart.

A contact impedance of a catheter electrode can be measured as a complex quantity (with magnitude and phase) or only as the magnitude (i. e., the amount) of the complex impedance. For example, in an embodiment, the wall contact detection subsystem comprises a measurement device being configured to determine a complex impedance between each of the catheter electrodes on the one side and a reference electrode on the other side. Alternatively, only an amount or a phase of the complex impedance between each of the catheter electrodes on the one side and the reference electrode on the other side may be determined by the measurement device.

In an embodiment, the wall contact detection subsystem is configured to determine that a catheter electrode is in contact with the heart chamber wall based on a determination that the complex impedance is smaller than a predetermined value. For example, when one of the catheter electrodes comes into contact with the endocardium, the measured impedance decreases. If the impedance of a catheter electrode has fallen below a certain value, it can be safely assumed that the respective electrode touches the heart wall.

In addition or alternatively, the contact detection subsystem may evaluate a change of a phase of a complex impedance to assess an electrode-endocardial contact. For example, a ratio between a resistance and a reactance changes when the wall contact is made and, which is reflected in the phase change.

In an embodiment, the localization electrodes of the position determination subsystem comprise at least 4 surface electrodes being configured to be attached on the patient's torso (e.g. in a tetraeder like positioning). In another embodiment, the localization electrodes of the position determination subsystem comprise at least 6 surface electrodes. For example, 4 or more surface electrodes may be attached on the chest and 4 or more other surface electrodes may be attached on the back of the patient (in total a minimum of 8 surface electrodes). In this way, an accurate localization of the catheter electrodes is possible.

The position determination subsystem may be configured to measure at least one of a voltage and a phase of a complex impedance at each of the localization electrodes. Further, the position determination subsystem may also be configured to measure an amount of a complex impedance and/or a reactance at each of the localization electrodes.

In an embodiment, the modelling system comprises a computer with an application software. For example, the modelling subsystem may be configured to create the model of the heart chamber geometry in the form of a virtual three-dimensional point cloud, wherein each point corresponds to a position from said set of positions of the catheter electrodes.

In a variant, the modeling subsystem is further configured to create a visual representation of the virtual three-dimensional point cloud, wherein said selected positions, which correspond to a determined contact with the heart chamber wall, are represented in a different type (e.g. color, symbol, blinking etc.) than the other positions in said set of positions of the catheter electrodes. For example, this function may be supported by a dedicated application software, which automatically marks the points of the point cloud in color and thus visualizes their affiliation to the heart wall. The system may further comprise a display unit, such as a monitor, that is configured for displaying a visual representation of the model of the heart chamber geometry.

By clearly displaying to the user the points where the endocardium was touched by a catheter electrode, an increased reliability of the geometrical model may be achieved. For example, the user can use the visual information to explore certain (yet unexplored) areas in a targeted manner. Specifically, the user can use the catheter to specifically scan only areas within the heart chamber that have no or only a few validated points. It may thus be avoided that parts of the heart chamber wall that have already been explored are unnecessarily touched again. As a result, the user may capture the heart chamber geometry very time-effectively, reliably and conveniently even without supplementary information, e. g., from an RF generator (impedance), X-ray images or a force sensor. In other words, the need to additionally use images from other imaging procedures (CT, MRI, 3D angiography) may thus be eliminated. Advantageously, the eliminated need for X-ray images reduces the radiation exposure for the patient. In addition, the validated contact points provide unambiguous geometric information and the generated model is very realistic.

In a second aspect, the present disclosure relates to an electroanatomical mapping (EAM) system, such as a system that is configured to perform a contactless EAM or a contact EAM based on measurements of a cardiac electrical activity. EAM systems are, as such, well known in the art and will therefore not be explained in detail here.

According to the second aspect, an EAM system comprises a system in accordance with the first aspect of the disclosure and is configured to carry out an electroanatomical mapping based on said generated model of the heart chamber geometry. This is to say that the EAM may be carried out at least partially in dependence on the model of the heart chamber geometry as generated by means of the system described above. For example, the reliability of the electroanatomical mapping may thus be supported, e. g., by the fact that only the signals detected at low impedance points contribute to the mapping. For instance, a voltage map or an activation map may thus be rendered more accurate due to the reliable determination of the spatial positions of the signal acquisition points. For example, the contact to the heart wall can be used as an exclusion criterion for the signals contributing to the map. Only signals detected by electrodes with contact to the wall will be used unchanged for EAM. Signals from other electrodes may be used only after correction though appropriate algorithms.

The teaching according to the present disclosure may increase the reliability of a contactless electroanatomical mapping (acquired by capturing heart signals on the body surface) by providing information for an equalization during the recalculation of heart signals (the so-called inverse problem) through a safely established electrode-endocardial contact.

Further, a system capable of performing impedance measurements by feeding signals into multiple catheter electrodes and simultaneously measuring cardiac signals both intracardially and at the body surface can provide information about the transmission path of the signals between the heart and the electrodes at the body surface. This can be used to equalize the contactless electroanatomical mapping by recalculating surface signals. More specifically, the system may provide for a possibility to equalize the projection of the signals captured on the surface onto the endocardium. By analyzing the contact maps and the surface signals, information can be provided for training the inverse calculation algorithm. With the system according to the present disclosure, the synchronous acquisition of intracardiac and surface signals as well as more accurate contact maps may thus be combined to recalculate or estimate the parameters in signal transmission between the heart and the body surface.

In a third aspect, the present disclosure relates to a medical method for determining a heart chamber geometry which makes use of a system according to the first aspect. The method comprises the following steps:
a) acquiring, by means of the position determination subsystem, position information for each of the plurality of catheter electrodes;
b) acquiring, by means of the wall contact detection subsystem, information indicating whether a respective catheter electrode is in contact with the heart chamber wall in a respective position as determined by the position determination subsystem; and
c) creating, by means of the modelling subsystem, a model of the heart chamber geometry by selecting, among a set of positions of the catheter electrodes as acquired by means of the position determination subsystem, positions in which the catheter electrodes are in contact with the heart chamber wall, as determined by means of the wall contact detection subsystem.

This method can also help to improve electroanatomical mapping if used with a system according to the second aspect of the disclosure.

All embodiments of the described system(s) can be combined in any desired way and can be transferred in an analogous manner to the described method(s), and vice versa.

Further details will be explained with respect to exemplary embodiments and accompanying Figures. In the Figures:
- Fig. 1: shows a schematic and exemplary view of a system for determining a heart chamber geometry in accordance with one or more embodiments;
- Fig. 2: shows a schematic and exemplary view of a system for determining a heart chamber geometry in accordance with one or more embodiments; and
- Fig. 3: shows a schematic and exemplary representation of the method in the form of a block diagram.

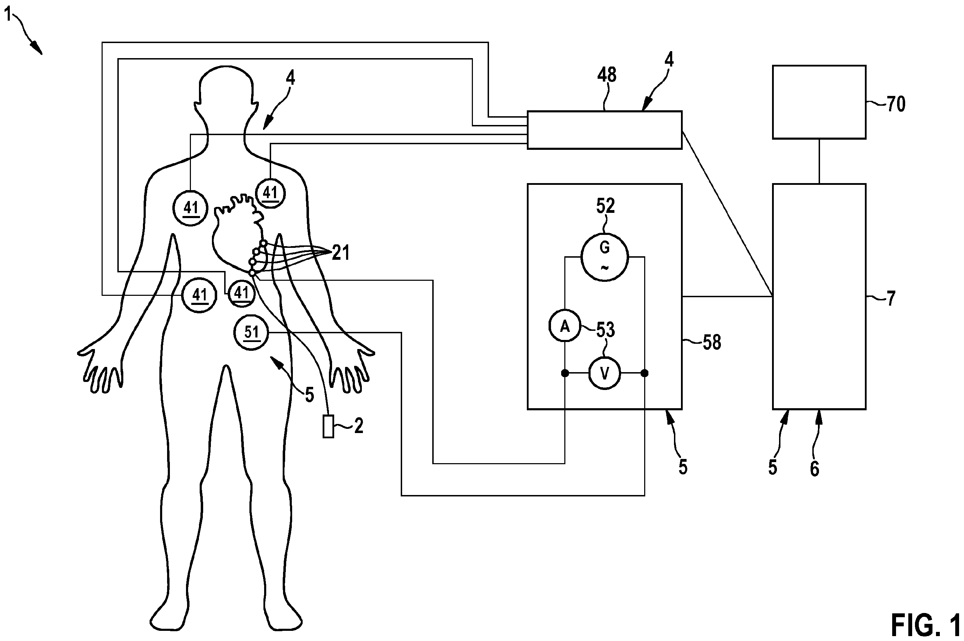

Fig. 1 schematically and exemplarily illustrates a system 1 for determining a heart chamber geometry (here: geometry of a ventricle). The system 1 comprises a catheter 2, which has been introduced in a cardiac ventricle of a patient. The catheter 2 has a plurality of catheter electrodes 21 attached in the vicinity of a distal end that is located inside the ventricle. For example, the catheter electrodes 21 may be configures as ring electrodes and/or tip electrodes. In Fig. 1, only 4 catheter electrodes 21 are shown as a schematic and exemplary illustration. In a preferred embodiment, 64 catheter electrodes are provided.

Further, the system 1 comprises a position determination subsystem 4 that is configured to determine a position of each of the catheter electrodes 21. To this end, the position determination subsystem 4 comprises multiple localization electrodes 41 in the form of surface electrodes, which are attached on the patient's torso, and an external device 48 in the form of a so-called surface satellite 48, which is connected to each of the surface electrodes 41. In the illustrated embodiment, 4 surface electrodes 41 are shown which are attached on a front portion of the patient's torso. In addition, 4 or more surface electrodes 41 may be attached on the back of the patient (not illustrated).

The position determination subsystem 4 may be configured to measure a voltage and/or a phase of a complex impedance at each of the localization electrodes 4. From the result of this measurement, a position of each of the catheter electrodes 21 may be determined. For example, to this end, an electrical probe signal may be fed to each of the catheter electrodes 21, as will be explained in further detail further below in connection with the wall contact detection subsystem 5.

The system 1 additionally includes a wall contact detection subsystem 5 that comprises an external device 58 in the form of a so-called intracardiac satellite 58. The wall contact detection subsystem 5 is configured to determine for each of the catheter electrodes 21 whether it is currently in contact with a ventricle wall of the patient's cardiac ventricle. To this end, the intracardiac satellite 58 comprises a signal generator 52 that is electrically coupled with the catheter electrodes 21. The signal generator 52 is configured to feed an electrical probe signal to each of the catheter electrodes 21. For example, the electrical probe signal may be a sinusoidal AC current with a frequency of approximately 25 kHz.

For example, up to 64 catheter electrodes 21 can be sequentially multiplexed with this signal at a multiplexing frequency of approximately 22 Hz. In other words, the signal generator 52 in this exemplary embodiment is configured to feed the electrical probe signal to the catheter electrodes 21 in a time multiplexed manner, such that each catheter electrode 21 receives the probe signal 22 times per second. This multiplexing frequency is sufficiently large so as to resolve the heartbeat of the patient in time. For example, this allows for the detection of an intermittent contact of a respective catheter electrode 21 with the ventricle wall, which results from the heartbeat.

The intracardiac satellite 58 is further equipped with one or more measurement devices 53, which are configured to measure a current of the electrical probe signal as well as voltage amplitudes between each of the catheter electrodes 21 and a reference electrode 51 of the wall contact detection subsystem 5. In the present example, the reference electrode 51 is a surface adhesive electrode 51. These measured values are then transmitted to a computer, e g. in the form of a PC (personal computer) 7, where the complex impedance between each catheter electrode 21 and the reference electrode 51 can be calculated. A complex impedance at each of the 64 catheter electrodes 21 may thus be determined 22 times per second. Localization measurements may be carried out simultaneously (i. e., also 22 times per second) by means of the surface electrodes 41 on each of the catheter electrodes 21. The results of the localization measurements are also transmitted from the surface satellite 48 to the PC 7. Thus, information on both the impedance of the catheter electrodes 21 and their position is available at the PC 7. The PC 7 is connected to a display unit 70 for showing the results.

In addition, intracardiac heart signals at the up to 64 catheter electrodes 21 may be recorded by the intracardiac satellite 58, and surface heart signals at 64 adhesive surface electrodes (not illustrated) may be recorded by the surface satellite 48.

It should be noted that, in the present exemplary embodiment, the PC 7 (including an application software running thereon) may form a part of both the position determination subsystem 4 and the contact wall detection subsystem 5. Thus, the subsystems 4, 5 need not be entirely separate subsystems, but may have common physical components. The same applies for the modelling subsystem 6 that is explained further below.

In its capacity as part of the wall contact detection subsystem 5, the PC 7 may be configured (e. g. via an appropriate application software) to determine for each of the catheter electrodes 21 whether the respective catheter electrode 21 is in contact with the ventricle wall. Specifically, it may be determined that a catheter electrode 21 is in contact with the ventricle wall if the complex impedance of the respective catheter electrode 21 is smaller than a predetermined value. When one of the catheter electrodes 21 comes into contact with the endocardium, the measured impedance decreases. If the impedance of a catheter electrode 21 has fallen below a certain value, it can thus be safely assumed that the respective electrode 21 touches the ventricle wall. In addition or alternatively, the wall contact detection subsystem 5 may evaluate a change of a phase of a complex impedance to assess an electrode-endocardial contact.

In the present embodiment, the PC 7 (including an appropriate application software being installed thereon) further fulfills the function of a modelling subsystem 6 that creates a 3D model of the heart chamber geometry by selecting, among a set of positions of the catheter electrodes as acquired by means of the position determination subsystem 4, positions in which the catheter electrodes 21 are in contact with the ventricle wall as determined by means of the wall contact detection subsystem 5.

For example, the PC 7 may thus create a visual representation of a virtual 3D point cloud, wherein said selected positions, which correspond to a determined contact of a located contact electrode 21 with the ventricle wall, are represented in a different color than the other positions in said set of positions of the located catheter electrodes 21.

Thus, as the user moves the catheter 2 within the ventricle to be examined, the application software running on the PC 7 can use the acquired localization signals to create the 3D model of the ventricle and color mark the points where the localized catheter electrodes 21 have palpated the endocardium.

The PC 7 of the illustrated embodiment comprises a display unit in the form of a monitor 10 for displaying this visual representation of the ventricle geometry to the user. As a result, the user can see in real time and with good accuracy which areas he has not yet explored and then guide the catheter 2 specifically to these locations.

For example, the system 1 described above may play a supporting role for an electroanatomical mapping (EAM) system (not illustrated), wherein a contact EAM or a contactless EAM may be carried out at least partially based on the generated 3D model of the heart chamber geometry. For example, such a system 1 may form a part of an EAM system.

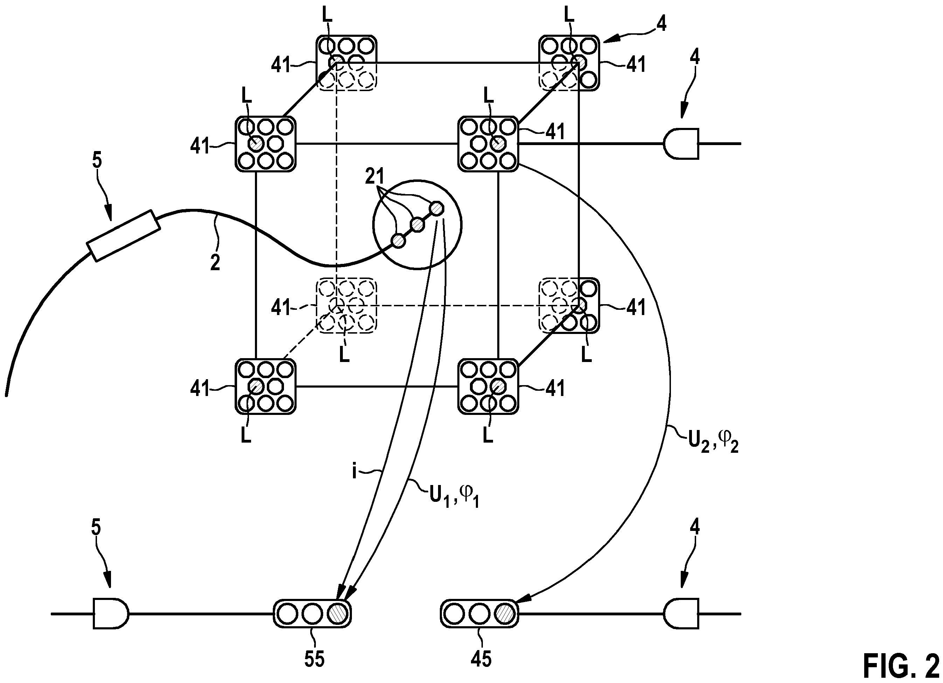

Fig. 2 schematically and exemplarily illustrates some components of a system 1 as described above in further detail. Generally, what has been stated above with respect to Fig. 1 also applies to the embodiment of Fig. 2. For example, a catheter 2 having a number (e. g. 64) of catheter electrodes 21 is shown, wherein the catheter 2 is introduced in a heart chamber (here ventricle), which is schematically depicted as a circle in Fig. 2. In addition, 8 localization electrodes 41 are shown, wherein, for example, the 4 localization electrodes 41 depicted in the front may be attached to a front portion of the patient's torso, and the 4 localization electrodes 41 depicted in the back may be attached to the patient's back.

As indicated in Fig. 2, a current i may be fed into the catheter electrodes 21. The current i may flow from there into a first sink 55 of the intracardiac satellite 58, where the current i may be measured. Further, each of a first voltage U1 against the first sink 55 and a corresponding first phase difference ϕ1 may be measured by the intracardiac satellite. This information may serve for calculating a complex impedance and, on this basis, safely determining an electrode-ventricular contact, as has been explained above with reference to Fig. 1.

As further indicated in Fig. 2, the position determination subsystem 4 may measure each of a second voltage U2 against a second sink 45 of the surface satellite 48, and a corresponding second phase difference ϕ2. This information may be used for determining the positions of each of the plurality of catheter electrodes 21. The modelling subsystem (not illustrated in Fig. 2) may then generate a model of the ventricle geometry based on the acquired information on position and wall contact, as described above.

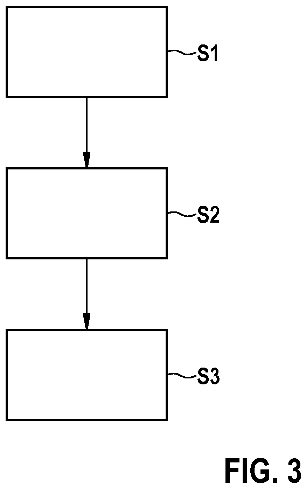

Fig. 3 schematically illustrates a method for determining a heart chamber geometry in the form of a block diagram. For example, the method may make use of the system 1 explained above with reference to Figs. 1 and 2.

In a first step S1, position information is acquired for each of the plurality of catheter electrodes 21 by means of the position determination subsystem 4.

In a second step S2, information is acquired for each catheter electrode 21 as to whether the respective catheter electrode is in contact with the heart chamber wall in a respective position as determined by the position determination subsystem 4. This step S2 may be carried out by means of the wall contact detection subsystem 5.

In a third step S3, a model of the heart chamber geometry is created by means of the modelling subsystem 6 by selecting, among a set of positions of the catheter electrodes 21 as acquired by means of the position determination subsystem 4, positions in which the catheter electrodes 21 are in contact with the heart chamber wall as determined by means of the wall contact detection subsystem 5.

In an analog manner, the geometry of an atrium can be determined.

For example, the method may be carried out in a computer-implemented manner by means of an appropriate application software.

### List of reference signs

- 1: System

- 2: Catheter
- 21: Catheter electrodes

- 4: Position determination subsystem
- 41: Localization electrodes
- 45: Second sink
- 48: Surface satellite

- 5: Wall contact detection subsystem
- 51: Reference electrode
- 52: Signal generator
- 53: Measurement device
- 55: First sink
- 58: Intracardiac satellite

- 6: Modelling subsystem

- 7: Personal computer
- 70: Display unit

- S1, S2, S3: Method steps

## Claims

1. A system (1) for determining a heart chamber geometry, comprising:
- a catheter (2) being configured to be introduced in a heart chamber of a patient, the catheter (2) comprising a plurality of catheter electrodes (21);
- a position determination subsystem (4) being configured to determine a position of each of the catheter electrodes (21) by means of multiple localization electrodes (41) that are configured to be arranged on the patient's body;
- a wall contact detection subsystem (5) being configured to determine for each of the catheter electrodes (21) whether the catheter electrode (21) is in contact with a heart chamber wall of the heart chamber by measuring at least one of a complex impedance, an amount of a complex impedance, and a phase of a complex impedance; and
- a modelling subsystem (6) being configured to create a model of the heart chamber geometry by selecting, among a set of positions of the catheter electrodes (21) as acquired by means of the position determination subsystem (4), positions in which the catheter electrodes (21) are in contact with the heart chamber wall as determined by means of the wall contact detection subsystem (5).

2. The system (1) of claim 1, wherein the wall contact detection subsystem (5) comprises a reference electrode (51) that is configured to be arranged on or inside the patient's body.

3. The system (1) of claim 2, wherein the wall contact detection subsystem (5) comprises a measurement device (53) being configured to determine at least one of a complex impedance between each of the catheter electrodes (21) and the reference electrode (51), an amount of a complex impedance between each of the catheter electrodes (21) and the reference electrode (51), and a phase of a complex impedance between each of the catheter electrodes (21) and the reference electrode (51).

4. The system (1) of one of the preceding claims, wherein the wall contact detection subsystem (5) comprises a signal generator (52) being configured to feed an electrical probe signal to each of the catheter electrodes (21).

5. The system (1) of claim 4, wherein the electrical probe signal is an alternating current signal having a frequency in the range from 10 kHz to 50 kHz.

6. The system (1) of claim 4 or claim 5, wherein the signal generator (52) is configured to sequentially feed the probe signal to the catheter electrodes (21) in a time multiplexed manner, such that each of the control electrodes (21) receives the probe signal at least 10 times per second.

7. The system (1) of one of the preceding claims, wherein the wall contact detection subsystem (5) is configured to determine that a catheter electrode (21) is in contact with the heart chamber wall based on at least one of:
- a determination that the complex impedance is smaller than a predetermined value; and
- a detection of a change of the phase of the complex impedance.

8. The system (1) of one of the preceding claims, wherein the catheter (2) comprises at least four catheter electrodes (21).

9. The system (1) of one of the preceding claims, wherein the localization electrodes (41) of the position determination subsystem (4) comprise at least four surface electrodes (41) being configured to be attached on the patient's torso.

10. The system (1) of one of the preceding claims, wherein the position determination subsystem (4) is configured to measure at least one of a voltage and a phase of complex impedance at each of the localization electrodes (41).

11. The system (1) of one of the preceding claims, wherein the modelling subsystem (6) is configured to create the model of the heart chamber geometry in the form of a virtual three-dimensional point cloud, wherein each point corresponds to a position from said set of positions of the catheter electrodes (21).

12. The system (1) of one of claim 11, wherein the modeling subsystem (6) is further configured to create a visual representation of the virtual three-dimensional point cloud, wherein said selected positions, which correspond to a determined contact with the heart chamber wall, are represented in a different type than the other positions in said set of positions of the catheter electrodes (21).

13. The system (1) of one of the preceding claims, wherein the system (1) further comprises a display unit (70) being configured for displaying a visual representation of the model of the heart chamber geometry.

14. An electroanatomical mapping system being configured for measuring and mapping a cardiac electrical activity, wherein the electroanatomical mapping system comprises the system (1) of one of the preceding claims, and wherein the electroanatomical mapping system is configured to carry out the mapping based on said model of the heart chamber geometry.

15. A method for determining a heart chamber geometry by means of a system (1) of one of the claims 1 to 13, the method comprising
- acquiring (S1), by means of the position determination subsystem (4), position information for each of the plurality of catheter electrodes (21);
- acquiring (S2), by means of the wall contact detection subsystem (5), information indicating whether a respective catheter electrode (21) is in contact with the heart chamber wall in a respective position as determined by the position determination subsystem (4); and
- creating (S3), by means of the modelling subsystem (6), a model of the heart chamber geometry by selecting, among a set of positions of the catheter electrodes (21) as acquired by means of the position determination subsystem (4), positions in which the catheter electrodes (21) are in contact with the heart chamber wall as determined by means of the wall contact detection subsystem (5).
